# FASCICULE DE BREVET EUROPEEN

(11) **EP 3 320 090 B1**
(45) Date de publication et mention de la délivrance du brevet: **23.11.2022**
(21) Numéro de dépôt: 16750925.6
(22) Date de dépôt: 06.07.2016
(51) Int. Cl.: C12N 9/26, C12P 19/22

(54) **PROCEDE DE FABRICATION DE MALTITOL PRESENTANT UN RENDEMENT AMELIORE**
VERFAHREN ZUR HERSTELLUNG VON MALTITOL MIT VERBESSERTER AUSBEUTE
METHOD OF MANUFACTURING MALTITOL WITH IMPROVED YIELD

(30) Priorité: 06.07.2015 FR 1556365
(43) Date de publication de la demande: 16.05.2018
(73) Titulaire: Roquette Frères, 62136 Lestrem (FR)
(72) Inventeur: LECOCQ, Aline, 59420 Mouvaux (FR); COURBOIS, Vincent, 62400 Bethune (FR)
(74) Mandataire: Plasseraud IP
(86) Numéro de dépôt international: PCT/FR2016/051704
(87) Numéro de publication internationale: WO 2017/006049

(56) Documents cités:
- WO-A1-2013/148152
- WO-A2-2013/114219
- WO-A2-2013/114223
- FR-A1- 2 905 705
- US-A- 4 024 000
- TOLAN J S ET AL: "Cellulase from Submerged Fermentation", ADVANCES IN BIOCHEMICAL ENGINEERING, BIOTECHNOLOGY, SPRINGER, BERLIN, DE, vol. 65, 4 mai 1999 (1999-05-04), pages 41-67, XP008122759, ISSN: 0724-6145, DOI: 10.1007/3-540-49194-5 [extrait le 2003-06-30]

## Description

### Domaine de l'invention

La présente invention est relative à un procédé de fabrication de maltitol obtenu à partir d'un sirop de maltose fabriqué par hydrolyse enzymatique d'un amidon à l'aide d'une solution aqueuse de beta-amylase stabilisée particulière. Ce procédé présente un rendement en maltitol amélioré.

### Etat de la technique

Le maltitol est un polyol qui présente un grand intérêt du fait qu'il est moins calorique que le saccharose, tout en possédant avantageusement des propriétés organoleptiques très voisines de celles de ce sucre ; de plus, il est également plus stable chimiquement que le saccharose. En outre, le maltitol possède la particularité d'être non cariogène, ce qui lui permet d'être utilisé dans de multiples applications dans l'industrie, notamment dans les industries alimentaires et pharmaceutiques.

Le maltitol est obtenu industriellement par une étape d'hydrogénation du maltose. On obtient généralement un sirop de maltitol à partir d'un sirop de maltose, la richesse dans le sirop de maltose soumis à l'étape d'hydrogénation déterminant la richesse en maltitol du sirop hydrogéné obtenu. Le sirop de maltitol peut être utilisé tel quel ; toutefois, il est généralement enrichi en maltitol en utilisant des techniques de fractionnement, notamment par chromatographie continue, ou encore en cristallisant le sirop hydrogéné, ce dernier ayant éventuellement été enrichi en maltitol préalablement.

En ce qui concerne la première étape de fabrication d'un sirop de maltose, elle peut comprendre au moins une étape d'hydrolyse d'un amidon granulaire, ledit procédé comprenant généralement une étape d'hydrolyse enzymatique utilisant une enzyme maltogénique. Cette enzyme peut être une beta-amylase.

Les beta-amylases sont des exo-hydrolases qui libèrent des unités maltose à partir des beta-extrémités non réductrices des polymères ou oligomères de glucose liés en alpha 1→4, la réaction s'arrêtant au premier point de branchement alpha 1→6 rencontré. Composants majoritaires du « pouvoir diastasique » (correspondant aux activités combinées des alpha-amylases, beta-amylases, alpha-glucosidases et enzymes débranchantes) durant le maltage (germination artificielle des graines de céréales), les activités beta-amylasiques isolées de ce cocktail enzymatique sont essentielles pour la production du maltose généré à partir d'amidon.

L'activité saccharifiante des seules beta-amylases est donc exploitée industriellement pour la production de maltose.

Ainsi, on comprend de ce qui précède que l'obtention d'un sirop à haute teneur en maltose est particulièrement avantageuse lorsque l'on souhaite fabriquer du maltitol. En effet, si l'on souhaite augmenter le rendement en maltitol d'un procédé, une des possibilités est d'augmenter la richesse en maltose dans le sirop produit par hydrolyse enzymatique de l'amidon.

C'est ainsi qu'il a été décrit dans de nombreux documents des procédés de fabrication de maltitol cherchant à obtenir des sirops présentant une richesse en maltose élevée.

A titre d'exemple, on peut citer la demande WO 2013/148152 qui décrit un procédé de fabrication d'un sirop riche en maltose à partir d'amidon granulaire comprenant une première étape de solubilisation d'amidon granulaire utilisant une alpha-amylase exogène pour former un mélange de dextrines et une seconde étape d'hydrolyse de ce mélange de dextrines à l'aide d'une enzyme maltogénique, qui peut être une beta-amylase, selon un ratio particulier alpha-amylase/enzyme maltogénique. D'autres procédés de fabrication de compositions riches en maltitol ont également été décrits dans les documents WO 2013/114223, WO 2013/114219 et FR 2 905 705.

Les procédés de fabrication de beta-amylases sont nombreux. On sait ainsi que les graines d'orge, de seigle ou de blé non germées sont autant de matériels biologiques de choix pour la préparation commerciale, à grande échelle, de beta-amylases. Il est par ailleurs connu de l'homme du métier que la moitié des beta-amylases extractibles des graines non germées peut être facilement obtenue sous la forme d'enzymes libres par extraction avec de l'eau et des solutions salines. L'autre moitié se présente en partie sous forme « liée » qui nécessite l'addition d'agents réducteurs ou des enzymes protéolytiques pour son extraction. Une autre fraction de beta-amylases non directement extractible, dite « latente » a également été décrite : il faut des détergents pour l'extraire des graines de céréales. Par ailleurs, les procédés d'extraction de la beta-amylase décrits dans l'état de la technique sont adaptés en fonction de l'application visée.

Dans les procédés industriels, après son extraction, la beta-amylase est systématiquement stockée avant d'être utilisée. Ce stockage peut durer au moins un jour, voire au moins une semaine, ou encore au moins un mois.

Or, il s'avère alors que l'activité enzymatique de la beta-amylase va diminuer au fil du temps. On rappelle que l'activité d'une enzyme est par définition la quantité de substrat transformé (ou de produit formé) par unité de temps et dans les conditions de fonctionnement optimales de l'enzyme (température, de pH ...). Cette grandeur quantifie donc l'efficacité de l'enzyme. Classiquement, la mesure de l'activité enzymatique est faite à travers la détermination d'un autre paramètre : l'activité diastasique. Cette dernière est exprimée en degré de pouvoir diastasique (°DP), défini comme la quantité d'enzyme contenue dans 0,1 ml d'une solution à 5 % en poids d'un échantillon de préparation d'enzymes suffisante pour réduire 5 ml de liqueur de Fehling, quand ledit échantillon est placé dans 100 ml du substrat pendant 1 h à 20°C.

On connaît aujourd'hui un certain nombre de documents décrivant des procédés d'obtention de beta-amylase, en vue d'améliorer leur stabilité du point de vue de leur activité enzymatique.

Le document CN102965358 divulgue un procédé d'obtention à partir du soja d'une beta-amylase par précipitation, puis drainage, clarification et ultrafiltration. Ledit procédé a recours à du chlorure de calcium, et optionnellement à des sels de l'acide sulfurique au niveau de l'étape de précipitation.

Le document CN102399763 décrit la production de beta-amylase à partir de sons, avec ajout de chlorure de calcium et d'hydrogène phosphate de sodium, puis concentration et stabilisation en présence de sorbitol et de sorbate de potassium, avant stérilisation.

Le document CN101544967 divulgue un procédé de fabrication de beta-amylases par précipitation, séparation et centrifugation, et préconise ensuite l'ajout de chlorure de calcium, d'acide orthophosphorique, de terres de diatomée et de glycérol.

Le document CN1225943 décrit un procédé de préparation de beta-amylase comprenant les étapes de d'ultrafiltration, concentration et précipitation d'extraits de poudre de soja, la précipitation étant précédée par l'ajout de sulfate de sodium et la régulation du pH entre 3,6 et 5.

Le document US 2,496,261 décrit un procédé d'obtention de beta-amylase à partir de la patate douce, qui comprend une étape de précipitation en présence de sulfate d'ammonium puis acidification avec de l'acide chlorhydrique.

Le document US 4,024,000 décrit un procédé de préparation de beta-amylase qui met en œuvre des ions divalents ou trivalents choisis parmi les hydroxydes de calcium, magnésium, baryum, aluminium et leurs sels, et régulation du pH dans un intervalle compris entre 4,5 et 8.

Or, force est de constater qu'aucune de ces solutions ne permet d'obtenir une préparation de beta-amylase sous forme d'une solution aqueuse qui soit suffisamment stable dans le temps pour maintenir son activité enzymatique de manière satisfaisante.

Poursuivant ses travaux, la Demanderesse est parvenue à démontrer qu'une sélection bien particulière d'additifs permettait d'atteindre un tel objectif. La solution aqueuse de beta-amylase stabilisée avec ces additifs présente ainsi, à durée de stockage équivalente, une meilleure activité enzymatique que les solutions aqueuses de beta-amylase déjà connues.

Ainsi, comme la beta-amylase est systématiquement stockée avant utilisation dans les procédés industriels, la Demanderesse est parvenue à un procédé de fabrication de maltitol permettant d'améliorer le rendement, dont une étape comprend une hydrolyse enzymatique d'amidon utilisant la solution aqueuse de beta-amylase stabilisée.

### Résumé de l'invention

L'invention a ainsi pour objet un procédé de fabrication de maltitol, comprenant :
a) une étape de production d'un sirop de maltose, dit « sirop de maltose A », par hydrolyse d'un amidon granulaire, cette étape comprenant un premier stade de liquéfaction dudit amidon granulaire par hydrolyse pour former un amidon liquéfié suivi d'un stade de saccharification de l'amidon liquéfié pour former le sirop de maltose A ;
b) éventuellement soumettre le sirop de maltose A à une étape de concentration, de dilution, d'enrichissement en maltose, et/ou de mélange avec un autre sirop de d'hydrolysat d'amidon pour former un autre sirop de maltose, dit « sirop de maltose B » ;
c) une étape d'hydrogénation du sirop de maltose A ou du sirop de maltose B pour former une composition aqueuse de maltitol, dite « composition de maltitol C » ;
d) éventuellement soumettre la composition de maltitol C à une étape de concentration, de dilution, d'enrichissement en maltitol et/ou de mélange avec un sirop de polyols additionnel pour former une composition aqueuse de maltitol, dite « composition de maltitol D » ;
e) éventuellement une étape de formation de poudre de maltitol à partir de la composition de maltitol C ou de la composition de maltitol D ;
f) une étape de récupération de la composition de maltitol C, de la composition de maltitol D ou de la poudre de maltitol ;
dans lequel le stade de saccharification de l'amidon liquéfié comprend l'introduction dans l'amidon liquéfié d'une solution aqueuse de beta-amylase comprenant en outre :
- de 0,05 à 0,5 % sorbate de potassium ;
- de 30 à 50 % glycérol ;
- de 0,05 à 0,5 % carbonate de sodium
ces pourcentages étant exprimés en pourcentage en poids sec de chaque constituant par rapport au poids total de ladite solution aqueuse.

De préférence, la solution aqueuse de beta-amylase stabilisée est stockée pendant un délai d'au moins un jour avant l'introduction dans l'amidon liquéfié, avantageusement dans un délai allant de 1 à 300 jours, éventuellement de 10 à 250 jours, notamment de 30 à 200 jours, par exemple de 60 à 150 jours.

En effet, ce procédé peut permettre d'obtenir, en comparaison avec des procédés similaires utilisant des solutions de beta-amylase déjà connues, des compositions présentant une richesse plus élevée en maltitol. Le procédé selon l'invention permet également de réduire, voire de supprimer, les étapes d'enrichissement en maltitol et/ou en maltose. Il peut également permettre de diminuer la durée de l'étape a). Le procédé selon l'invention permet d'obtenir au moins un de ces avantages, ceux-ci concourant à améliorer le rendement en maltitol de ce procédé.

### Description détaillée de l'invention

Comme décrit précédemment, le procédé selon l'invention concerne un procédé de fabrication de maltitol, dont une étape met en jeu l'hydrolyse enzymatique d'un amidon à l'aide d'une solution aqueuse de beta-amylase particulière.

La première étape du procédé selon l'invention comprend une étape a) de production d'un sirop de maltose par hydrolyse d'un amidon granulaire.

Le terme « amidon » comprend selon la présente invention tous les types d'amidons, ce qui inclut bien évidemment les fécules. L'amidon granulaire peut être de toute origine botanique et notamment peut provenir du maïs, de la pomme de terre, de la patate douce, du blé, du riz, du pois, de la fève, de la féverole, du manioc, du sorgho, du konjac, du seigle, du sarrasin et de l'orge, avantageusement du blé, du maïs, du pois ou de la pomme de terre.

Cette étape d'hydrolyse d'un amidon granulaire comprend un premier stade de liquéfaction dudit amidon granulaire par hydrolyse pour former un amidon liquéfié suivi d'un stade de saccharification de l'amidon liquéfié pour former le sirop de maltose.

Lors du stade de liquéfaction, cet amidon granulaire est généralement mis sous la forme d'un lait d'amidon, c'est-à-dire une suspension d'amidon granulaire dans l'eau. Ce lait d'amidon est généralement additionné d'acide dans le cas d'une liquéfaction dite acide, ou d'une enzyme dans le cas d'une liquéfaction enzymatique.

De préférence, le stade de liquéfaction d'amidon est réalisé par hydrolyse enzymatique à l'aide d'une alpha-amylase.

Dans le procédé conforme à l'invention, on préfère effectuer une hydrolyse ménagée du lait d'amidon de façon à obtenir un amidon liquéfié à faible taux de transformation. Ainsi, les conditions de température, de pH, de taux d'enzyme et de calcium, connues de l'homme du métier, sont déterminées de manière telles qu'elles permettent d'obtenir un amidon liquéfié de faible DE (Dextrose Equivalent), généralement inférieur à 10, de préférence inférieur à 7, par exemple inférieur à 4.

A titre d'exemple, le stade de liquéfaction peut être conduit en au moins deux sous-étapes, la première consistant à chauffer, pendant quelques minutes et à une température comprise entre 105 et 108°C, le lait d'amidon en présence d'une alpha-amylase (type TERMAMYL 120L commercialisée par la société Novozymes) et d'un activateur à base de calcium, la seconde sous-étape consistant à chauffer le lait d'amidon ainsi traité à une température comprise entre 95 et 100°C pendant une à deux heures.

Une fois l'étape de liquéfaction terminée et l'amidon liquéfié obtenu, dans les conditions de teneur en matières sèches, de pH, de taux d'enzyme et de calcium bien connues de l'homme du métier, on peut procéder à l'inhibition de l'alpha-amylase. Cette inhibition de l'alpha-amylase peut se faire de préférence par voie thermique, en procédant en sortie de liquéfaction à un choc thermique de quelques secondes à une température supérieure ou égale à 130°C.

Après le premier stade de liquéfaction dudit amidon granulaire, on soumet l'amidon liquéfié obtenu à un stade de saccharification pour former le sirop de maltose.

Selon l'invention, le stade de saccharification de l'amidon liquéfié comprend l'introduction dans l'amidon liquéfié d'une solution aqueuse de beta-amylase comprenant en outre :
- de 0,05 à 0,5 % sorbate de potassium ;
- de 30 à 50 %glycérol ;
- de 0,05 à 0,5 %carbonate de sodium,
ces pourcentages étant exprimés en poids sec de chaque constituant par rapport au poids total de ladite solution aqueuse de beta-amylase.

La solution aqueuse de beta-amylase utile à cette étape peut avantageusement comprendre :
a) de 0,1 à 0,3 %, et très préférentiellement environ 0,2 % de sorbate de potassium ;
b) de 35 à 45 %, et très préférentiellement environ 40 % de glycérol ;
c) de 0,1 à 0,3 %, et très préférentiellement environ 0,2 % de carbonate de sodium ;
ces pourcentages étant exprimés en poids sec de chaque constituant par rapport au poids total de ladite solution aqueuse de beta-amylase.

La solution aqueuse de beta-amylase utile à l'invention comprend avantageusement, par rapport à son poids total, un poids sec de beta-amylase compris dans la gamme allant de 5 à 20 %, préférentiellement de 10 à 20 %, très préférentiellement environ 15 % de son poids total.

Même si la solution aqueuse de beta-amylase utile à l'invention peut éventuellement comprendre d'autres constituants que l'eau et les constituants décrits ci-dessus, selon un mode de réalisation, elle consiste en un mélange :
- de 0,05 à 0,5 % en poids, préférentiellement de 0,1 à 0,3 %, et très préférentiellement environ 0,2 % de sorbate de potassium ;
- de 30 à 50 % en poids, préférentiellement de 35 à 45 %, et très préférentiellement environ 40 % de glycérol ;
- de 0,05 à 0,5 % en poids, préférentiellement de 0,1 à 0,3 %, et très préférentiellement environ 0,2 % de carbonate de sodium ;
- de 5 à 20 % en poids, préférentiellement de 10 à 20 %, très préférentiellement environ 15 % de beta-amylase ;
- de 35 à 50% en poids, préférentiellement de 40 à 45 % en eau.

La solution aqueuse de beta-amylase présente une excellente stabilité, ce qui lui permet de maintenir son activité enzymatique au cours du temps. Ainsi, le procédé selon l'invention peut comprendre une étape de stockage, qui peut se faire à température ambiante ou dans des conditions thermostatées, pendant un délai d'au moins un jour avant l'introduction dans l'amidon liquéfié, avantageusement dans un délai allant de 1 à 300 jours, éventuellement de 10 à 250 jours, notamment de 30 à 200 jours, par exemple de 60 à 150 jours.

Ce délai est défini comme le délai compris entre la fin de la fabrication de beta-amylase stabilisée et le moment de son introduction dans l'amidon liquéfié lors du procédé.

La solution aqueuse de beta-amylase utile à l'invention peut être fabriquée par simple mélange des additifs (sorbate de potassium, glycérol et carbonate de sodium) avec la beta-amylase, par exemple en les introduisant et mélangeant dans une solution de beta-amylase aqueuse non stabilisée, le mélange pouvant se faire très simplement à température ambiante.

Le sorbate de potassium, le glycérol et le carbonate de sodium sont préférentiellement sous forme de solutions aqueuses. L'homme du métier saura adapter l'extrait sec de ces solutions, par rapport à la solubilité des produits mais aussi de manière à limiter la viscosité de ces solutions et de façon à le rendre aisément manipulable et notamment pompable.

Par exemple, la beta-amylase peut être obtenue sous la forme d'une solution aqueuse de beta-amylase non stabilisée par les étapes consistant à :
- fournir une fraction soluble de plantes amidonnières ;
- réaliser sur ladite fraction soluble une étape de microfiltration afin d'obtenir un perméat de microfiltration ;
- réaliser sur le perméat de microfiltration une étape d'ultrafiltration afin d'obtenir un rétentat d'ultrafiltration.

C'est donc ledit rétentat d'ultrafiltration qui constitue généralement ladite solution aqueuse de beta-amylase non stabilisée, dans laquelle peuvent être introduits le sorbate de potassium, le glycérol et le carbonate de sodium.

Par ailleurs, il est souhaitable de maintenir ladite solution de beta-amylase telle qu'obtenue à une température inférieure à 15°C, préférentiellement inférieure à 10°C, idéalement aux environs de 5°C, de manière à améliorer encore le maintien de son activité enzymatique.

Il est également souhaitable de préparer la solution aqueuse utile à l'invention le plus rapidement possible après obtention de la solution de beta-amylase non stabilisée afin d'en améliorer encore l'activité enzymatique, de préférence dans un délai inférieur à 1 jour.

Selon l'invention, le stade de saccharification de l'amidon liquéfié peut se faire de manière classique, hormis que celui-ci est réalisé à l'aide de la solution stabilisée de beta-amylase décrite précédemment.

Le stade de saccharification peut se faire en présence d'au moins une enzyme additionnelle, cette enzyme étant par exemple choisie parmi les alpha-amylases maltogéniques, les alpha-amylases fongiques et/ou les enzymes débranchantes.

Les ajouts d'enzymes peuvent se faire en une seule fois ou en plusieurs fois, simultanément.

A titre d'exemple d'alpha-amylase maltogénique, on peut citer celle commercialisée par la société Novozymes, sous le nom Maltogénase 5.

L'enzyme débranchante peut être choisie dans le groupe constitué par les pullulanases et les isoamylases. La pullulanase est, par exemple, celle commercialisée par la société ABM sous la dénomination PULLUZYMER. L'isoamylase est, par exemple, celle commercialisée par la société HAYASHIBARA.

Le ou les enzymes additionnelles peuvent être introduites dans le lait d'amidon liquéfié simultanément, avant ou après la solution stabilisée de beta-amylase.

Par exemple, on peut tout d'abord réaliser un ajout d'alpha-amylase maltogénique, puis un ajout de la solution stabilisée de beta-amylase. Dans ce cas, si on ajoute une enzyme débranchante, l'ajout peut se faire au moment de l'ajout de l'alpha-amylase maltogénique, au moment de l'ajout de la beta-amylase ou encore ultérieurement.

Lors du stade de saccharification, il est possible de suivre la teneur des différents hexoses, et de faire évoluer la proportion des teneurs en sélectionnant les enzymes additionnelles et en ajustant les quantités d'enzymes.

Selon un premier mode de réalisation, on introduit la solution de beta-amylase stabilisée au même moment que la ou les enzyme(s) additionnelle(s).

Selon un deuxième mode de réalisation, on soumet tout d'abord le lait d'amidon liquéfié à l'action d'une alpha-amylase maltogénique. Lors de cette première étape de saccharification, l'alpha-amylase maltogénique peut être ajoutée en une seule fois ou en plusieurs fois. On poursuit ensuite, après avoir laissé agir l'alpha-amylase maltogénique, la saccharification du lait d'amidon liquéfié au moyen de la solution de beta-amylase stabilisée décrite précédemment.

De manière alternative, on peut selon un troisième mode de réalisation soumettre le lait d'amidon liquéfié à l'action de la solution de beta-amylase stabilisée décrite précédemment. Lors de cette première étape de saccharification, la solution de beta-amylase stabilisée peut être ajoutée en une seule fois ou en plusieurs fois. On poursuit ensuite, après avoir laissé agir la solution de beta-amylase stabilisée, la saccharification du lait d'amidon liquéfié au moyen d'une alpha-amylase maltogénique.

On peut également associer aux enzymes ayant une activité maltogénique (alpha-amylase maltogénique et beta-amylase) une enzyme hydrolysant spécifiquement les liaisons alpha-1,6 de l'amidon, également appelée «enzyme débranchante ». Cet ajout d'une enzyme débranchante permet d'une part d'accélérer les réactions d'hydrolyse sans simultanément accélérer les réactions de réversion et, d'autre part, de réduire la quantité d'oligosaccharides hautement branchés résistant normalement à l'action des enzymes maltogéniques. Cette enzyme débranchante peut notamment être une pullulanase ou une isoamylase. Avantageusement, le stade de saccharification se fait en présence d'isoamylase pour laquelle la société demanderesse a constaté qu'elle permettait non seulement d'obtenir un sirop de maltose présentant une teneur en maltose plus élevée qu'en utilisant une pullulanase, mais aussi d'obtenir un sirop de maltose présentant une teneur réduite en maltosyl-1,6 maltose (et donc en maltosyl-1,6 maltitol après hydrogénation).

L'hydrolysat ainsi saccharifié est ensuite généralement filtré, par exemple en utilisant un filtre à pré-couche ou une microfiltration sur membranes. L'hydrolysat peut être également déminéralisé.

Selon l'invention, on obtient à l'issue de l'étape a), un sirop de maltose A.

Le procédé comprend éventuellement une étape b) dans laquelle on soumet le sirop de maltose A à une étape de concentration, de dilution, d'enrichissement en maltose, et/ou de mélange avec un sirop d'hydrolysat d'amidon additionnel pour former une composition aqueuse de maltose B. Une étape de concentration du sirop consiste à augmenter sa matière sèche par évaporation partielle de l'eau présente dans le sirop tandis qu'une étape de dilution consiste à diminuer la matière sèche par ajout d'eau.

Il est éventuellement possible d'enrichir le sirop en maltose, notamment en utilisant un tamisage moléculaire. Cette étape de tamisage moléculaire peut permettre ainsi de récupérer :
- soit une première fraction enrichie en maltose et oligosaccharides supérieurs et une seconde fraction enrichie en glucose ;
- soit une première fraction enrichie en oligosaccharides supérieurs et une seconde fraction enrichie en maltose et glucose ;
- soit, enfin, une première fraction enrichie en oligosaccharides supérieurs, une deuxième fraction enrichie en en maltose et une troisième fraction enrichie en glucose.

Cette étape de tamisage moléculaire peut consister, par exemple, en une étape de séparation chromatographique ou en une étape de séparation sur membranes.

L'étape de fractionnement chromatographique est effectuée de manière connue en soi, de façon discontinue ou continue (lit mobile simulé), sur des adsorbants du type résines cationiques, ou sur des zéolithes fortement acides, chargées préférentiellement à l'aide d'ions alcalins ou plus préférentiellement à l'aide d'ions sodium.

En lieu et place de l'étape de séparation chromatographique, il est possible de mettre en œuvre une étape de séparation par nanofiltration sur membranes. Des membranes de différents diamètres de pores sont fabriquées à partir de nombreux polymères et copolymères du type polysulfones, polyamides, polyacrylonitrates, polycarbonates ou polyfuranes.

Des exemples de l'utilisation de telles membranes sont décrits notamment dans les documents US-A-4.511.654, US-A-4.429.122 et WO-A-95/10627.

Il est également possible de mélanger le sirop de maltose A avec un sirop d'hydrolysat d'amidon additionnel, ce sirop de d'hydrolysat d'amidon additionnel pouvant comprendre du glucose, du maltose et/ou d'autres oses.

Selon le procédé de l'invention, il est tout à fait possible lors de l'étape b) de combiner au moins deux étapes choisies parmi les étapes de concentration, de dilution, d'enrichissement en maltose et de mélange avec un sirop de d'hydrolysat d'amidon additionnel ; en d'autres termes, il est tout à fait possible par exemple de réaliser une étape de concentration du sirop de maltose A suivie d'une étape d'enrichissement en maltose pour former un sirop de maltose B.

Les sirops de maltose A et B peuvent avoir, selon les conditions, une teneur en maltose variable. Le sirop peut comprendre, par rapport à sa masse sèche, au moins 30 % en maltose, par exemple de 45 à 99,9%, notamment de 50 à 99%.

Le procédé selon l'invention comprend également une étape c) d'hydrogénation du sirop de maltose A ou du sirop de maltose B pour former une composition aqueuse de maltitol C.

Le sirop de maltose peut être facilement hydrogéné catalytiquement. L'hydrogénation d'un tel sirop s'effectue conformément aux règles de l'art, qui conduisent par exemple à la production de maltitol à partir du maltose et de sorbitol à partir du glucose.

On peut utiliser pour cette étape aussi bien des catalyseurs à base de ruthénium que des catalyseurs au nickel de RANEY. On préfère cependant utiliser des catalyseurs au nickel de RANEY qui sont moins onéreux.

Dans la pratique, on utilise généralement de 1 à 10 % en poids de catalyseur par rapport à la matière sèche du sirop de maltose soumis à l'hydrogénation. L'hydrogénation s'effectue de préférence sur un sirop de maltose dont la matière sèche est comprise entre 15 et 55 %, dans la pratique voisine de 30 à 50 %, sous une pression d'hydrogène comprise entre 20 et 200 bars. Elle peut être effectuée de manière continue ou discontinue. Lorsque l'on opère de manière discontinue, la pression d'hydrogène utilisée peut généralement être comprise entre 30 et 60 bars. La température à laquelle se déroule l'hydrogénation est généralement comprise entre 100 et 150°C. On veille généralement aussi à maintenir le pH du milieu d'hydrogénation par l'addition de soude ou de carbonate de soude par exemple, mais sans dépasser un pH de 9,0. Cette manière de faire permet d'éviter l'apparition de produits de cracking ou d'isomérisation.

La réaction peut être arrêtée lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine généralement le catalyseur par filtration et peut réaliser une déminéralisation sur des résines cationiques et anioniques.

A l'issue de l'étape c), on obtient une composition aqueuse de maltitol C, cette composition comprenant généralement différents polyols dont le maltitol. La teneur dans les différents polyols dépend principalement de la composition dans les différents oses du sirop de maltose soumis à hydrogénation.

Le procédé comprend éventuellement une étape d) dans laquelle on soumet la composition aqueuse de maltitol C à une étape de concentration, de dilution, d'enrichissement en maltitol et/ou de mélange avec un sirop de polyols additionnel.

Les méthodes de concentration, de dilution etd'enrichissement décrites pour l'étape b) peuvent être utilisées également en vue de réaliser l'étape d). Le sirop de polyols additionnel peut notamment comprendre du sorbitol, du maltitol et/ou d'autres polyols. Selon le procédé de l'invention, il est tout à fait possible lors de l'étape d) de combiner au moins deux étapes choisies parmi les étapes de concentration, de dilution, d'enrichissement en maltitol et de mélange avec un sirop de polyols additionnel ; en d'autres termes, il est tout à fait possible par exemple de réaliser une étape de concentration de la composition aqueuse de maltitol C suivie d'une étape d'enrichissement en maltitol.

Selon le procédé de l'invention, il est également possible de réaliser une étape e) de formation de poudre de maltitol à partir de la composition de maltitol C ou de la composition de maltitol D, par les méthodes classiquement utilisées. Cette étape e) peut être réalisée par une étape de cristallisation, éventuellement combinée avec une étape de texturation de la poudre ainsi cristallisée.

A titre d'exemple de méthodes de fabrication de poudre de maltitol, on peut citer celles décrites dans les documents EP 2055197 A1, WO 2009112740 A2, EP2093231 A1, EP 905138 A1 et EP 735042 A1.

Le procédé selon l'invention comprend une étape de récupération f), étape dans laquelle on récupère la composition de maltitol C obtenue à l'issue de l'étape c), la composition de maltitol D obtenue à l'issue de l'étape d), ou la poudre de maltitol obtenue à l'étape e).

Selon la variante où le maltitol est sous la forme d'une composition aqueuse, la quantité massique en maltitol sec de la composition aqueuse, exprimée en masse sèche, va avantageusement de 45 à 99,9%, par exemple de 50 à 99%. De préférence, la matière sèche de la composition aqueuse va de 50 à 95%, notamment de 70 à 90%.

Les exemples qui suivent permettent de mieux appréhender l'invention, sans toutefois en limiter la portée.

### EXEMPLES

### Fabrication de solutions aqueuses de beta amylase

On commence par prélever en amidonnerie de blé une fraction soluble à l'entrée de l'évaporateur des solubles, étape classiquement mise en œuvre pour fabriquer des produits destinés à l'alimentation du bétail, une fois concentrés. Ces produits sont commercialisés par la société Demanderesse sous le nom de Corami^{®}. Ces fractions solubles présentent un pH compris entre 4 et 5 et une activité beta-amylasique de l'ordre de 30 °DP / mL.

On réalise ici, sur un équipement à l'échelle pilote, la microfiltration de fractions solubles de blé. L'unité de microfiltration est équipée de membranes céramiques en oxyde de titane, dont le seuil de coupure est égal à 0,2 µm. Le débit de perméat est fixé à 12 L/(h m²). Le facteur de concentration volumique est égal à 1,5. La température et le pH du perméat sont respectivement égaux à 45°C et environ 4,5.

On ajoute dans la fraction soluble de la protéase Neutrase 0,8L (Novozyme), à une concentration fixée à 0,1 % en volume par rapport au volume total de ladite composition. On laisse au préalable agir cette protéase pendant 1 heure à température ambiante.

Puis on réalise une ultrafiltration telle que décrite ci-avant.

On obtient un perméat de microfiltration avec un degré DP de 25 °DP / ml après 1 heure de microfiltration, ce degré reflétant l'activité enzymatique de la solution contenant la β-amylase. La mesure de l'activité enzymatique est déterminée par l'activité diastasique. Cette dernière est exprimée en degré de pouvoir diastasique (°DP), défini comme la quantité d'enzyme contenue dans 0,1 ml d'une solution à 5 % en poids d'un échantillon de préparation d'enzymes suffisante pour réduire 5 ml de liqueur de Fehling, quand ledit échantillon est placé dans 100 ml du substrat pendant 1 h à 20°C.

L'étape de microfiltration est suivie par une étape d'ultrafiltration, réalisée sur le perméat de microfiltration. Elle a pour principal objectif de concentrer ledit perméat et de le débarrasser d'éventuels sels résiduels contaminants, sucres et protéines. Le pilote d'ultrafiltration est équipé de membranes organiques en polysulfone, ayant un seuil de coupure 25 KDa (membranes Alfa Laval). La température de filtration est fixée à 25°C pour limiter au maximum les développements bactériologiques et préserver l'activité enzymatique. La Pression TransMembranaire (PTM) est fixée à 4 bars maximum.

On obtient alors une solution aqueuse de beta-amylase qui consiste en le rétentat d'ultrafiltration, ayant une teneur en poids sec de beta-amylase égale à 15 % de son poids total.

Différents cocktails, tel qu'indiqué dans les tableaux 1 à 3, ont été testés. Tous les % sont exprimés en % en poids sec de produit, par rapport au poids total de la solution aqueuse. Une fois les préparations réalisées, un dosage enzymatique de chaque échantillon (contenu en pot de 100 ml stériles) est effectué selon la méthode décrite dans la demande de brevet FR 2 994 440 (mesure de l'activité beta-amylasique). Cette valeur fait objet de référence pour l'ensemble de l'étude. Les différents échantillons sont ensuite placés dans une étuve à température contrôlée : 37°C pendant la période souhaité ; un prélèvement pour mesure de l'activité beta-amylasique résiduelle est alors effectué à différents moments (les jours de prélèvement sont indiqués dans les tableaux 1 à 3). Les résultats figurent dans les tableaux 1 à 3, et sont exprimés comme des % d'activité beta-amylasique résiduelle. On choisit de se placer à 37°C de manière à accélérer les phénomènes qui président à la chute de l'activité enzymatique.

Le tableau 1 bis démontre que le meilleur résultat est obtenu avec le mélange de 40 % de glycérol, 0,2 % de sorbate de potassium et 0,2 % de Na₂CO₃. Il démontre également que, par rapport à d'autres cocktails mettant en œuvre d'autres ingrédients, c'est bien la solution selon l'invention qui permet de développer le meilleur niveau de stabilité. Il s'agit donc bien d'une sélection non évidente d'ingrédients pour réaliser un cocktail, qui conduit à des résultats surprenants et tout à fait avantageux, en termes de limitation de la perte d'activité enzymatique. Le tableau 1 bis démontre que des cocktails comme décrits dans la revendication 1 de la présente demande permettent de développer des niveaux de stabilité très importants. Quelles que soient les quantités en constituants du cocktail, la stabilité est toujours au moins légèrement supérieure, voire bien supérieure. La plus grande stabilité est par ailleurs obtenue avec le dernier cocktail décrit dans ce tableau, réalisé avec les doses optimales de chaque ingrédient, comme décrit dans la revendication 2 de la présente demande.

Le tableau 2 démontre que le glycérol, utilisé seul, et même à forte dose, ne permet pas d'atteindre le niveau de stabilité satisfaisant. Le tableau 3 démontre que la substitution du glycérol par des sucres ne permet pas elle non plus d'atteindre un niveau de stabilité satisfaisant.

**Tableau 1**

| jours | 50% glycérol + 0,2 % SP | 50% glycérol + 0,2 % SP + 1% Na₂HPO₄ | 40% glycérol + 0,2 % SP + 1% Na₂CO₃ | 40% sorbitol + 0,2 % SP + 1% Na₂HPO₄ | 40% sorbitol + 0,2 % SP + 1% CaCO₃ | 50% glycérol + 0,2 % SP + 1% CaCO₃ |
|---|---|---|---|---|---|---|
| 0 | 100 | 100 | 100 | 100 | 100 | 100 |
| 20 | 98 | 98 | | | | |
| 30 | | | 72 | | | |
| 34 | 89 | 89 | | 77 | 85 | 92 |
| 60 | | | 60 | | | |
| 72 | 48 | 70 | 75 | 66 | 69 | 70 |
| 90 | | 45 | 50 | 44 | 45 | 47 |

**Tableau 1 bis**

| jours | 60% glycérol + 0,2 % SP + 0,4% Na₂CO₃ | 40% glycérol + 1 % SP + 0,4% Na₂CO₃ | 40% glycérol + 0,2 % SP + 0,4% Na₂CO₃ | 40% glycérol + 0,4 % SP + 0,2% Na₂CO₃ | 40% glycérol + 0,2 % SP + 0,2% Na₂CO₃ |
|---|---|---|---|---|---|
| 0 | 100 | 100 | 100 | 100 | 100 |
| 72 | 74 | 73 | 76 | 76 | 80 |
| 90 | 49 | 48 | 54 | 54 | 60 |

**Tableau 2***

| | 0% glycérol | 30% glycérol | 40% glycérol | 50% glycérol |
|---|---|---|---|---|
| 0 | 100 | 100 | 100 | 100 |
| 30 | 0 | 53 | 65 | 69 |
| 60 | 0 | 27 | 44 | 56 |
| 90 | 0 | 7 | 16 | 28 |

**Tableau 3**

| jours | 50% glucose | 10% glycerol + 30% glucose | 20% glycerol + 20% glucose | 40% glucose + 0,5% Na₂HPO₄ | 40% glucose + 3% NaCl | 40% maltose | 40% mélange (45% glucose, 10% fructose, 45% maltose) |
|---|---|---|---|---|---|---|---|
| 0 | 100 | 100 | 100 | 100 | 100 | 100 | 100 |
| 30 | 66 | 53 | 61 | 82 | 42 | 46 | 39 |
| 60 | 43 | 35 | 37 | 43 | 15 | 30 | 20 |
| 90 | 28 | 22 | 22 | 22 | 7 | | |

| | | | | | | | |
|---|---|---|---|---|---|---|---|
| SP : sorbate de potassium * On note par ailleurs la formation d'un dépôt insoluble important dans le cas du carbonate de calcium | | | | | | | |

### Fabrication de sirops de maltose

On réalise ensuite 4 essais, portant sur la fabrication de sirops de maltose à partir de 4 solutions aqueuses de beta-amylase stabilisées. 3 essais selon l'invention et 1 essai de référence sont réalisés en utilisant des solutions stabilisées ayant été stockées 90 jours à 25 °C avant d'être utilisées.

Un lait d'amidon, à une matière sèche de 31 %, est liquéfié de manière classique à l'aide de 0,2 % d'une alpha-amylase (TERMAMYL120L commercialisée par la société NOVOZYMES) à un pH de 5,7 à 6,5 jusqu'à un DE environ égal à 6.

On chauffe ensuite le milieu réactionnel pendant quelques secondes à 140 °C de manière à inhiber l'alpha-amylase, puis on ajuste le pH entre 5 et 5,5 et la température à 55 °C.

La saccharification est conduite à une matière sèche de 35 %, ou légèrement inférieure, en présence de pullulanase (PULLUZYME 750L commercialisée par la société ABM) et d'alpha-amylase maltogénique (MALTOGENASE 4000L commercialisée par la société NOVOZYMES) et d'une solution aqueuse de beta-amylase, à des doses égales à 0,1 % sur matière sèche.

La solution aqueuse de beta-amylase consiste en le rétentat d'ultrafiltration, ayant une teneur en poids sec de beta-amylase égale à 15 % de son poids total, comme décrit dans l'exemple précédent.

Dans un premier test hors invention (CP), cette solution a été stabilisée avec le cocktail selon la deuxième colonne du tableau 1 (50% glycérol + 0,2 % SP + 1% Na₂HPO₄). La solution est restée à une température de 25°C pendant 90 jours avant d'être utilisée comme indiquée ci-dessus.

Dans un deuxième test selon l'invention (EX1), cette solution a été stabilisée avec le cocktail selon la dernière colonne du tableau 1 bis (40% glycérol + 0,2 % SP + 0,2% Na₂CO₃). La solution est restée à une température de 25°C pendant 90 jours avant d'être utilisée comme indiquée ci-dessus.

Dans un troisième test selon l'invention (EX2), cette solution a été stabilisée avec le cocktail selon la quatrième colonne du tableau 1 bis (40% glycérol + 0,4 % SP + 0,2% Na₂CO₃). La solution est restée à une température de 25°C pendant 90 jours avant d'être utilisée comme indiquée ci-dessus.

Dans un quatrième test selon l'invention (EX3), cette solution a été stabilisée avec le cocktail selon la troisième colonne du tableau 1 (40% glycérol + 0,2 % SP + 1% Na₂CO₃). La solution est restée à une température de 25°C pendant 90 jours avant d'être utilisée comme indiquée ci-dessus.

Pour ces tests, la saccharification, qui dure environ 72 heures, donne un sirop de maltose montrant pour chacun des exemples les compositions suivantes :
Sirop de maltose CP :
   glucose : 2 %, maltose : 77,9 % maltotriose : 5,6 %
Sirop de maltose EX1 :
   glucose : 5 %, maltose : 88 % maltotriose : < 1,5 %
Sirop de maltose EX2 :
   glucose : 3,2 % - maltose : 82,1 % - maltotriose: 3,7 %
Sirop de maltose EX3 :
   glucose : 2,8 % - maltose : 81 % - maltotriose : 4,6 %

### Fabrication de maltitol

Les sirops de maltose fabriqués précédemment (CP, EX1, EX2, EX3) sont amenés à 45% de matière sèche dans un hydrogénateur de 18 m³. La température est amenée à 140°C et la pression d'hydrogène à 60 bars. Le pH diminue lentement jusqu'à une valeur de 4,5. A ce moment, le pH est remonté à 8 par ajout de soude. Quand la teneur en sucres réducteurs est inférieure à 0,1% sur sec, la réaction est arrêtée.

La composition aqueuse de maltitol ainsi obtenue est ensuite purifiée par déminéralisation et traitement sur charbon actif. Cette composition est alors évaporée à 70% de matière sèche. On obtient ainsi une composition aqueuse de maltitol présentant un taux de sucres réducteurs totaux égal à environ 0,2%.

Pour chacune des compositions, les pourcentages massiques en sorbitol, maltitol et maltotriitol, exprimés en matière sèche, sont reportées dans le Tableau ci-dessous.

| Polyol | CP | EX1 | EX2 | EX3 |
|---|---|---|---|---|
| % sorbitol | 4 | 7 | 5 | 5 |
| % maltitol | 76 | 86 | 80 | 79 |
| % maltotriitol | 5 | 1,5 | 3,5 | 4 |

Il apparaît clairement des pourcentages ci-dessus qu'il est possible, à partir du procédé selon l'invention, d'améliorer la richesse des compositions aqueuses de maltitol. Ceci permet d'obtenir un excellent rendement en maltitol du procédé, supérieur à celui obtenu à partir d'un procédé utilisant dans les mêmes conditions (stockage...) une beta-amylase stabilisée autrement que par la combinaison de stabilisants utile à l'invention.

## Revendications

1. - Procédé de fabrication de maltitol, comprenant :
a) une étape de production d'un sirop de maltose, dit « sirop de maltose A », par hydrolyse d'un amidon granulaire, cette étape comprenant un premier stade de liquéfaction dudit amidon granulaire par hydrolyse pour former un amidon liquéfié suivi d'un stade de saccharification de l'amidon liquéfié pour former le sirop de maltose A ;
b) éventuellement soumettre le sirop de maltose A à une étape de concentration, de dilution, d'enrichissement en maltose, et/ou de mélange avec un autre sirop de d'hydrolysat d'amidon pour former un autre sirop de maltose, dit « sirop de maltose B » ;
c) une étape d'hydrogénation du sirop de maltose A ou du sirop de maltose B pour former une composition aqueuse de maltitol, dite « composition de maltitol C » ;
d) éventuellement soumettre la composition de maltitol C à une étape de concentration, de dilution, d'enrichissement en maltitol et/ou de mélange avec un sirop de polyols additionnel pour former une composition aqueuse de maltitol, dite « composition de maltitol D » ;
e) éventuellement une étape de formation de poudre de maltitol à partir de la composition de maltitol C ou de la composition de maltitol D ;
f) une étape de récupération de la composition de maltitol C, de la composition de maltitol D ou de la poudre de maltitol ;
dans lequel le stade de saccharification de l'amidon liquéfié comprend l'introduction dans l'amidon liquéfié d'une solution aqueuse de beta-amylase comprenant en outre :
• de 0,05 à 0,5 % de sorbate de potassium ;
• de 30 à 50 % de glycérol ;
• de 0,05 à 0,5 % de carbonate de sodium ;
ces pourcentages étant exprimés en pourcentage en poids sec de chaque constituant par rapport au poids total de ladite solution aqueuse.

2. - Procédé de fabrication de maltitol selon la revendication 1, comprenant :
a) une étape de production d'un sirop de maltose, dit « sirop de maltose A », par hydrolyse d'un amidon granulaire, cette étape comprenant un premier stade de liquéfaction dudit amidon granulaire par hydrolyse pour former un amidon liquéfié suivi d'un stade de saccharification de l'amidon liquéfié pour former le sirop de maltose A ;
b) éventuellement soumettre le sirop de maltose A à une étape de concentration, de dilution, d'enrichissement en maltose, et/ou de mélange avec un autre sirop de d'hydrolysat d'amidon pour former un autre sirop de maltose, dit « sirop de maltose B » ;
c) une étape d'hydrogénation du sirop de maltose A ou du sirop de maltose B pour former une composition aqueuse de maltitol, dite « composition de maltitol C » ;
d) éventuellement soumettre la composition de maltitol C à une étape de concentration, de dilution, d'enrichissement en maltitol et/ou de mélange avec un sirop de polyols additionnel pour former une composition aqueuse de maltitol, dite « composition de maltitol D » ;
e) éventuellement une étape de formation de poudre de maltitol à partir de la composition de maltitol C ou de la composition de maltitol D ;
f) une étape de récupération de la composition de maltitol C, de la composition de maltitol D ou de la poudre de maltitol ;
dans lequel le stade de saccharification de l'amidon liquéfié comprend l'introduction dans l'amidon liquéfié d'une solution aqueuse de beta-amylase comprenant en outre :
• du sorbate de potassium ;
• du glycérol ;
• du carbonate de sodium.
et **caractérisé en ce que** la solution aqueuse de beta-amylase stabilisée est stockée pendant un délai d'au moins un jour avant l'introduction dans l'amidon liquéfié, avantageusement dans un délai allant de 1 à 300 jours, éventuellement de 10 à 250 jours, notamment de 30 à 200 jours, par exemple de 60 à 150 jours.

3. - Procédé selon l'une des revendications précédentes, **caractérisé en ce que** la solution aqueuse de beta-amylase comprend :
a) de 0,1 à 0,3 %, et préférentiellement environ 0,2 % de sorbate de potassium ;
b) de 35 à 45 %, et préférentiellement environ 40 % de glycérol ;
c) de 0,1 à 0,3 %, et préférentiellement environ 0,2 % de carbonate de sodium ;
ces pourcentages étant exprimés en pourcentage en poids sec de chaque constituant par rapport au poids total de ladite solution aqueuse.

4. - Procédé selon une des revendications précédentes, **caractérisé en ce que** la solution aqueuse présente une teneur en poids sec de beta-amylase comprise entre 5 et 20 %, préférentiellement entre 10 et 20 %, très préférentiellement environ 15 % de son poids total.

5. - Procédé selon l'une des revendications précédentes **caractérisé en ce que** le stade de liquéfaction d'amidon est réalisé par hydrolyse enzymatique à l'aide d'une alpha-amylase.

6. - Procédé selon l'une des revendications précédentes, **caractérisé en ce que** le stade de saccharification se fait en présence d'au moins une enzyme additionnelle, cette enzyme pouvant être choisie parmi les alpha-amylases maltogéniques, les alpha-amylases fongiques et/ou les enzymes débranchantes.

7. - Procédé selon l'une des revendications précédentes **caractérisé en ce que** le maltitol récupéré à l'étape f) est sous la forme d'une composition aqueuse et **en ce que** la quantité massique en maltitol de la composition aqueuse, exprimée en masse sèche, va de 30 à 99,9%, par exemple de 50 à 99%.

## Patentansprüche

1. Verfahren zur Herstellung von Maltitol, umfassend:
a) einen Schritt der Herstellung eines Maltosesirups, der als "Maltosesirup A" bezeichnet wird, durch Hydrolyse einer körnigen Stärke, wobei dieser Schritt eine erste Stufe der Verflüssigung der körnigen Stärke durch Hydrolyse umfasst, um eine verflüssigte Stärke zu bilden, gefolgt von einer Stufe der Verzuckerung der verflüssigten Stärke, um den Maltosesirup A zu bilden;
b) gegebenenfalls Konzentrieren, Verdünnen, Anreichern mit Maltose und/oder Mischen des Maltosesirups A mit einem anderen Stärkehydrolysatsirup, um einen anderen Maltosesirup zu bilden, der als "Maltosesirup B" bezeichnet wird;
c) einen Schritt der Hydrierung des Maltosesirups A oder des Maltosesirups B, um eine wässrige Maltitol-Zusammensetzung zu bilden, die als "Maltitol-Zusammensetzung C" bezeichnet wird;
d) gegebenenfalls Konzentrieren, Verdünnen, Anreichern mit Maltitol und/oder Mischen der Maltitol-Zusammensetzung C mit einem zusätzlichen Polyolsirup, um eine wässrige Maltitol-Zusammensetzung zu bilden, die als "Maltitol-Zusammensetzung D" bezeichnet wird;
e) gegebenenfalls einen Schritt zur Bildung von Maltitolpulver aus der Maltitol-Zusammensetzung C oder der Maltitol-Zusammensetzung D;
f) einen Schritt der Rückgewinnung der Maltitol-Zusammensetzung C, der Maltitol-Zusammensetzung D oder des Maltitol-Pulvers,
wobei die Stufe der Verzuckerung der verflüssigten Stärke das Einbringen einer wässrigen Beta-Amylase-Lösung in die verflüssigte Stärke umfasst, die ferner umfasst:
• 0,05 bis 0,5 % Kaliumsorbat;
• 30 bis 50 % Glycerol;
• 0,05 bis 0,5 % Natriumcarbonat;
wobei diese Prozentsätze als Trockengewichtsprozente der einzelnen Bestandteile in Bezug auf das Gesamtgewicht der wässrigen Lösung ausgedrückt sind.

2. Verfahren zur Herstellung von Maltitol nach Anspruch 1, umfassend:
a) einen Schritt der Herstellung eines Maltosesirups, der als "Maltosesirup A" bezeichnet wird, durch Hydrolyse einer körnigen Stärke, wobei dieser Schritt eine erste Stufe der Verflüssigung der körnigen Stärke durch Hydrolyse umfasst, um eine verflüssigte Stärke zu bilden, gefolgt von einer Stufe der Verzuckerung der verflüssigten Stärke, um den Maltosesirup A zu bilden;
b) gegebenenfalls Konzentrieren, Verdünnen, Anreichern mit Maltose und/oder Mischen des Maltosesirups A mit einem anderen Stärkehydrolysatsirup, um einen anderen Maltosesirup zu bilden, der als "Maltosesirup B" bezeichnet wird;
c) einen Schritt der Hydrierung des Maltosesirups A oder des Maltosesirups B, um eine wässrige Maltitol-Zusammensetzung zu bilden, die als "Maltitol-Zusammensetzung C" bezeichnet wird;
d) gegebenenfalls Konzentrieren, Verdünnen, Anreichern mit Maltitol und/oder Mischen der Maltitol-Zusammensetzung C mit einem zusätzlichen Polyolsirup, um eine wässrige Maltitol-Zusammensetzung zu bilden, die als "Maltitol-Zusammensetzung D" bezeichnet wird;
e) gegebenenfalls einen Schritt zur Bildung von Maltitolpulver aus der Maltitol-Zusammensetzung C oder der Maltitol-Zusammensetzung D;
f) einen Schritt der Rückgewinnung der Maltitol-Zusammensetzung C, der Maltitol-Zusammensetzung D oder des Maltitol-Pulvers;
wobei die Stufe der Verzuckerung der verflüssigten Stärke das Einbringen einer wässrigen Beta-Amylase-Lösung in die verflüssigte Stärke umfasst, die weiterhin umfasst:
• Kaliumsorbat;
• Glycerol;
• Natriumcarbonat,
und **dadurch gekennzeichnet, dass** die stabilisierte wässrige Beta-Amylase-Lösung für einen Zeitraum von mindestens einem Tag vor dem Einbringen in die Flüssigstärke gelagert wird, vorzugsweise innerhalb eines Zeitraums von 1 bis 300 Tagen, gegebenenfalls 10 bis 250 Tagen, insbesondere 30 bis 200 Tagen, z. B. 60 bis 150 Tagen.

3. Verfahren nach einem der vorhergehenden Ansprüche, **dadurch gekennzeichnet, dass** die wässrige Beta-Amylase-Lösung umfasst:
a) 0,1 bis 0,3 %, vorzugsweise etwa 0,2 %, Kaliumsorbat;
b) 35 bis 45 % und vorzugsweise etwa 40 % Glycerol;
c) 0,1 bis 0,3 % und vorzugsweise etwa 0,2 % Natriumcarbonat;
wobei diese Prozentsätze als Trockengewichtsprozente der einzelnen Bestandteile in Bezug auf das Gesamtgewicht der wässrigen Lösung ausgedrückt sind.

4. Verfahren nach einem der vorhergehenden Ansprüche, **dadurch gekennzeichnet, dass** die wässrige Lösung einen Trockengewichtsgehalt an Beta-Amylase zwischen 5 und 20 %, vorzugsweise zwischen 10 und 20 %, stark bevorzugt von etwa 15 % ihres Gesamtgewichts aufweist.

5. Verfahren nach einem der vorhergehenden Ansprüche, **dadurch gekennzeichnet, dass** die Stufe der Stärkeverflüssigung durch enzymatische Hydrolyse mit Hilfe einer alpha-Amylase durchgeführt wird.

6. Verfahren nach einem der vorhergehenden Ansprüche, **dadurch gekennzeichnet, dass** die Stufe der Verzuckerung in Gegenwart wenigstens eines zusätzlichen Enzyms durchgeführt wird, wobei dieses Enzym aus maltogenen alpha-Amylasen, Pilz-alpha-Amylasen und/oder abzweigenden Enzymen ausgewählt werden kann.

7. Verfahren nach einem der vorhergehenden Ansprüche, **dadurch gekennzeichnet, dass** das in Schritt f) gewonnene Maltitol in Form einer wässrigen Zusammensetzung vorliegt und dass die Maltitol-Massemenge der wässrigen Zusammensetzung, ausgedrückt als Trockenmasse, im Bereich von 30 bis 99,9 %, z. B. 50 bis 99 %, liegt.

## Claims

1. - A process for producing maltitol, comprising:
a) a step of producing a maltose syrup, referred to as "maltose syrup A", by hydrolysis of a granular starch, this step comprising a first stage of liquefaction of said granular starch by hydrolysis to form a liquefied starch, followed by a stage of saccharification of the liquefied starch to form the maltose syrup A;
b) optionally subjecting the maltose syrup A to a step of concentration, of dilution, of enrichment in maltose, and/or of mixing with another starch hydrolyzate syrup to form another maltose syrup, referred to as "maltose syrup B";
c) a step of hydrogenation of the maltose syrup A or of the maltose syrup B to form an aqueous composition of maltitol, referred to as "maltitol composition C";
d) optionally subjecting the maltitol composition C to a step of concentration, of dilution, of enrichment in maltitol, and/or of mixing with an additional polyol syrup to form an aqueous maltitol composition, referred to as "maltitol composition D";
e) optionally a step of formation of maltitol powder from the maltitol composition C or from the maltitol composition D;
f) a step of recovering the maltitol composition C, the maltitol composition D or the maltitol powder;
wherein the stage of saccharification of the liquefied starch comprises the introduction, into the liquefied starch, of an aqueous solution of beta-amylase, also comprising:
• from 0.05 to 0.5% of potassium sorbate;
• from 30 to 50% of glycerol;
• from 0.05 to 0.5% of sodium carbonate
these percentages being expressed as percentage by dry weight of each constituent relative to the total weight of said aqueous solution.

2. - A process for producing maltitol as claimed in claim 1, comprising:
a) a step of producing a maltose syrup, referred to as "maltose syrup A", by hydrolysis of a granular starch, this step comprising a first stage of liquefaction of said granular starch by hydrolysis to form a liquefied starch, followed by a stage of saccharification of the liquefied starch to form the maltose syrup A;
b) optionally subjecting the maltose syrup A to a step of concentration, of dilution, of enrichment in maltose, and/or of mixing with another starch hydrolyzate syrup to form another maltose syrup, referred to as "maltose syrup B";
c) a step of hydrogenation of the maltose syrup A or of the maltose syrup B to form an aqueous composition of maltitol, referred to as "maltitol composition C";
d) optionally subjecting the maltitol composition C to a step of concentration, of dilution, of enrichment in maltitol, and/or of mixing with an additional polyol syrup to form an aqueous maltitol composition, referred to as "maltitol composition D";
e) optionally a step of formation of maltitol powder from the maltitol composition C or from the maltitol composition D;
f) a step of recovering the maltitol composition C, the maltitol composition D or the maltitol powder;
wherein the stage of saccharification of the liquefied starch comprises the introduction, into the liquefied starch, of an aqueous solution of beta-amylase, also comprising:
• potassium sorbate;
• glycerol;
• sodium carbonate
wherein the stabilized beta-amylase aqueous solution is stored for a time of at least one day before introduction into the liquefied starch, advantageously for a time ranging from 1 to 300 days, optionally from 10 to 250 days, especially from 30 to 200 days, for example from 60 to 150 days.

3. - The process as claimed in the preceding claims, **characterized in that** the aqueous solution of beta-amylase comprises:
a) from 0.1 to 0.3%, and preferentially approximately 0.2%, of potassium sorbate;
b) from 35 to 45%, and preferentially approximately 40%, of glycerol;
c) from 0.1 to 0.3%, and preferentially approximately 0.2%, of sodium carbonate;
these percentages being expressed as percentage by dry weight of each constituent relative to the total weight of said aqueous solution.

4. - The process as claimed in one of the preceding claims, **characterized in that** the aqueous solution has a content by dry weight of beta-amylase of between 5 and 20%, preferentially between 10 and 20%, very preferentially approximately 15% of the total weight thereof.

5. - The process as claimed in one of the preceding claims, **characterized in that** the stage of starch liquefaction is carried out by enzymatic hydrolysis by means of an alpha-amylase.

6. - The process as claimed in one of the preceding claims, **characterized in that** the stage of saccharification is carried out in the presence of at least one additional enzyme, this enzyme being able to be selected from maltogenic alpha-amylases, fungal alpha-amylases and/or debranching enzymes.

7. - The process as claimed in one of the preceding claims, **characterized in that** the maltitol recovered in step f) is in the form of an aqueous composition and **in that** the amount by weight of maltitol of the aqueous composition, expressed as dry weight, ranges from 30 to 99.9%, for example from 50 to 99%.
